# EUROPÄISCHE PATENTSCHRIFT

(11) **EP 0 599 153 B1**
(45) Veröffentlichungstag und Bekanntmachung des Hinweises auf die Patenterteilung: **23.12.1998**
(21) Anmeldenummer: 93118326.3
(22) Anmeldetag: 12.11.1993
(51) Int. Cl.: G01N 21/71, G01N 33/44

(54) **Verfahren und Vorrichtung zur stofflichen Identifikation von Werkstoffen**
Method and device for the identification of materials
Méthode et appareil pour l'identification de matériaux

(30) Priorität: 21.11.1992 DE 4239479
(43) Veröffentlichungstag der Anmeldung: 01.06.1994
(73) Patentinhaber: LASER ZENTRUM HANNOVER e.V., D-30419 Hannover (DE)
(72) Erfinder: Burmester, I., Dipl.-Chem., D-30165 Hannover (DE); Engel, K., Dipl.-Ing., D-30453 Hannover (DE)
(74) Vertreter: König, Norbert, Dipl.-Phys. Dr.

(56) Entgegenhaltungen:
- EP-A- 0 497 397
- DE-A- 3 735 286
- US-A- 4 928 254

## Beschreibung

Die Erfindung betrifft ein Verfahren und eine Vorrichtung zur stofflichen Identifikation von Werkstoffen, wobei die Werkstoffe mit Laserenergie beaufschlagt werden und die Wärmeabstrahlung der Werkstoffe nach Beendigung der Erwärmung erfaßt und werkstoffspezifisch hinsichtlich optischer und thermischer Materialeigenschaften ausgewertet wird.

Im Zusammenhang mit dem Werkstoffrecycling stellt sich das Problem der möglichst automatischen Selektion unterschiedlicher nicht gemeinsam wiederverwertbarer Werkstoffe. Für die Wiederverwertbarkeit der Werkstoffe ist die Sortenreinheit der aufzuarbeitenden Werkstoffe von entscheidender Bedeutung, da mit zunehmender Vermischung der Gebrauchswert der Werkstoffe drastisch abnimmt.

Bei der Erkennung und Trennung von verschiedenen Kunststoffsorten finden zum heutigen Zeitpunkt häufig manuelle Sortierverfahren Einsatz. Die Unwirtschaftlichkeit und die mangelnde Leistungsfähigkeit einer manuellen Trennung bedingen den beschränkten Einsatz lediglich für die Sortierung von größeren, unzerkleinerten Kunststoffteilen, die zum Teil bei der Automobilverwertung anfallen. Für die Sortierung von kleineren Kunststoffteilen, die im Hausmüll- und Verpackungsmüllbereich anfallen, ist dieses Verfahren nicht geeignet, vgl. D. Braun, Deutsches Kunststoffinstitut "Einfache Methoden zur Kunststofferkennung, Vortrag beim DVM Tag 1992 "Bauteil 1992", Berlin. Für das Recycling kleinerer Kunststoffteile muß die Verwendung einer automatischen Kunststofferkennung gekoppelt mit einer nachfolgenden automatischen Trennung erfolgen, vgl. Sortenreines Recycling setzt eindeutig Produktbezeichnung voraus, Österreich. Kunstst. Zeitung 1990, Ausgabe 21 (5/6), S. 113-115.

Derzeit existieren zwei automatische Trennverfahren, die in begrenztem Umfang und auch nur für einige Kunststoffe eingesetzt werden.

Ein Trennverfahren, was z. Z. auch im technischen Maßstab eingesetzt wird, ist die Separation der Kunststoffe nach ihrer spezifischen Dichte. Hierbei werden sie in Leicht- und in eine Schwerfraktion unterteilt (G. Hörber, G. Ropertz, P. Kaniut "Das AKW-Kunststoffaufbereitungssystem - eine moderne und wirtschaftliche Alternative für die Wiederverwertung von "Alt"-Kunststoffen, in Aufbereitungs-Technik" (1989), Asg. 30(8), S. 500-506; A. Ansems, "Kunststoffe aus Abfällen. Trennung an der Quelle bestimmt die Wiederverwendungsmöglichkeiten", in Zeitschr. Kunstst. Rubber (1988), Ausg. 41(12), S. 62-69; A. Funke, J. Noack, "Homogenisieren von Sekundärpolyolefinen", in Zeitschr. Plaste Kautschuk (1987), Ausg. 34(2), S. 72-73). Der Leichtfraktion gehören die Polyolefine an, die als Polyethylen und Polypropylen einer weiteren Trennung bedürfen, da sie gemischt nicht wieder verarbeitbar sind. Die Schwerfraktion enthält alle übrigen Kunststoffsorten und ist ohne weitere Separation ebenfalls nicht für die Wiederverarbeitung geeignet. Eine weitere Auftrennung dieser Fraktionen kann über die spezifische Dichte nur in Verbindung mit hohen Sortenverunreinigungen erfolgen. Die technische Durchführung wird bei diesem Prinzip zum einen mit Hilfe eines Hydrozyklons und zum anderen in einer Schwimm-Sink-Rinne in Verbindung mit Wasser als Trennflüssigkeit bewirkt.

Das andere technisch eingesetzte Verfahrensprinzip der Flotation liegt darin begründet, daß sich an die hydrophobe Oberfläche von Kunststoffen selektiv Luftblasen anlagern, wodurch diese Stoffe auf Wasser aufschwimmen. Durch gezielte Zugabe von Reagenzien kann man die Anlagerung von Luftblasen an bestimmte Kunststoffe unterbinden (E. Kroker, F+E Vorhaben, Pilotvorhaben zum Trennen und Aufbereiten Kunststoffen aus Haushalts und Gewerbeabfällen, Auskünfte der Amtlichen Material Prüfanstalt, Hannover 1989). Die Nachteile des Verfahrens liegen zum einen in der begrenzten Auftrennmöglichkeit in nur zwei Kunststofffraktionen pro Anwendung und zum anderen in den möglichen Beeinflussungen der Kunststoffe durch den Kontakt mit den Trennungsreagenzien. Ferner lassen sich, bedingt durch die oberflächenspezifische Trennung, nur unverschmutzte sowie nicht lackierte und unbeschichtete Kunststoffe separieren, was eine weitere erhebliche Einschränkung des Verfahrens darstellt.

Es sind weitere Methoden zur Erkennung der Kunststoffsorte vorgeschlagen worden, die aber von einer umfassenden Lösung des Sortier-Problems noch weit entfernt sind, und eine technische Anwendung des jeweiligen Verfahrens kann heute weitgehend ausgeschlossen werden.

Ein Prinzip der Kunststofferkennung basiert auf einer Identifikation der Kunststoffe durch einen schon bei der Herstellung des Kunststoffes beigemischten Fluoreszenzfarbstoff (K. Luttermann, A. Becker, Bayer AG, Kennzeichnung von Kunststoffen mit Fluoreszenzfarbstoffen, Vortrag beim DVM Tag 1992 "Bauteil 1992", Berlin). Hierbei wird durch Einwirkung von Licht durch das Auftreten von Fluoreszenzstrahlung eine schnelle und auch sichere Erkennung des Kunststoffes möglich. Allerdings müßten sich alle Kunststoffhersteller über eine einheitliche und sortenspezifische Beimischung der Fluoreszenzfarben schon bei der Herstellung der Kunststoffe verständigen. Die Problematik liegt somit in der Durchsetzung der notwendigen generellen Einführung und Normierung dieser Beimischung von Fluoreszenzfarben.

Ein ähnliches Prinzip wird bei der Röntgenfluoreszenzanalyse verwirklicht, wobei hier kunststoffspezifische Dotierelemente oder Zuschlagstoffe bei der Herstellung beigemischt werden müssen, um eine spätere Erkennung zu ermöglichen (K. H. Folkerts, Vortrag im 384. Dechema-Kolloquim, 30.1.1992, Frankfurt/Main).

Eine weitere Methode stellt die Kunststofferkennung unter Verwendung eines AOTF-Reflexionsspektrometers dar (N. Eisenreich, Vortrag im 384. Dechema-Kolloquim, 30.1.1992, Frankfurt/Main; N. Eisenreich, H. Krause, H. Kull, Th. Messer, Schnelle Erkennung von Kunststoffen mit AOTF-Reflexionsspektrometer, VDI Bereichte Nr. 934, 1991, S. 447-459), bei der das NIR-Reflexionsspektrum für die Identifikation der Kunststoffsorte Verwendung findet. Hierbei enthält das Reflexionsspektrum des nahen Infrarotfrequenzbereichs (1000 bis 2500 nm) ausreichend Information, um im Fingerprintbereich den untersuchten Kunststoff erkennen zu können. Problematisch und technisch aufwendig ist die schnelle Auswertung der sehr komplizierten Spektren, die sich zusätzlich in Abhängigkeit von zugesetzten Additiven, Farbstoffen und verschiedenen Zusatzstoffen stark verändern. Probleme treten weiterhin bei der Untersuchung von ver- schmutzten und gefüllten Kunststoffproben auf. Dieses Verfahren ist technisch sehr aufwendig.

Es ist auch schon vorgeschlagen worden zur Identifikation der Kunststoffe, die Produkte des thermischen Abbaues von Kunststoffen mit Hilfe der Massenspektroskopie zu untersuchen (H. Müller von der Haegen, Vortrag im 384. Dechema-Kolloquim, 30.1.1992, Frankfurt/Main). Bei diesem Verfahren würde man somit die Kunststoffe nur indirekt über die sortenspezifischen Pyrolyseprodukte erkennen können. Probleme treten hier beim Einschleusen der gasförmigen Produkte in das Hochvakuum eines Massenspektrometers auf. Zusätzlich ist der hohe Zeitaufwand der Kombination aus thermischer Zersetzung, MS-Analyse und nachfolgender Auswertung zu berücksichtigen. Weiterhin sind die Pyrolyseprodukte von verschiedenen Kunststoffsorten teilweise identisch, so daß eine Trennung ebenfalls erschwert ist.

Die Verwendung von Trennflüssigkeiten zur Sortierung von Kunststoffgemischen anhand der spezifischen Dichte ist ebenfalls Gegenstand von Untersuchungen gewesen (U. Leuning, Kunststoffrecycling in Italien. Ziel der Regierung is eine Rücklaufquote bei Kunststoffverpackungen von 40 %, Zeitschr. Umwelt, Ausg. 20(4), 1990, S. 164-166). Auch hier konnte aufgrund der ähnlichen spezifischen Dichten keine ausreichende Unterscheidung der verschiedenen Kunststoffsorten getroffen werden.

In den USA ist eine elektromagnetische Trennmethode (R. T. Gottesmann, Vortrag IUPAC International Symposium Recycling of Polymers, Marbella, Spanien 18.-20. September 1991) entwickelt worden, bei der aber ausschließlich PVC von Polyolefinen abgetrennt werden kann und somit auch hier keine Lösung des generellen Trennproblems bei gemischt anfallenden Kunststoffen zu erwarten ist.

Zusammenfassend ist daher festzustellen, daß die bekannten Methoden keine technische Lösung für die Wiederverwertung von Kunststoffgemischen darstellen.

Aus der DE 37 35 286 A1 ist ein Verfahren zur Bestimmung der Zusammensetzung von mineralischen Lagerstätten bekannt. Bei diesem Verfahren wird die gesamte Oberfläche einer Lagerstätte, deren Zusammensetzung bestimmt werden soll, mit einem Laserstrahl rasterförmig abgetastet und die dabei eintretende Temperaturerhöhung von einem Infrarot-Sensor empfangen und gemessen.

Aus der US 4 928 254 ist eine Vorrichtung zur genauen und zerstörungsfreien Messung der Wärmeleitfähigkeit bekannt, die einen Laser zur Beaufschlagung einer dünnen Probe mit Laserenergie sowie eine Infrarot-Diode aufweist, die auf der dem Laser abgewandten Seite der Probe angeordnet ist und zur Detektion des Aufheizvorganges der Probe dient.

Aus der EP 0 497 397 A1 ist ein Verfahren zur qualitativen Analyse von Kunststoffteilchen bekannt, bei dem ein zu analysierendes Kunststoffteilchen durch einen UV-Laser mit Laserenergie beaufschlagt wird, so daß sich auf der Oberfläche des zu analysierenden Kunststoffteilchens ein Plasma bildet, das im UV-Bereich spektroskopisch analysiert wird.

Die Aufgabe der vorliegenden Erfindung besteht darin, ein Verfahren und eine Vorrichtung zur schnellen und zuverlässigen Identifikation verschiedener Werkstoffe anzugeben.

Diese Aufgabe wird verfahrensmäßig durch die Ausbildung gemäß Kennzeichen des Anspruchs 1 gelöst. Vorteilhafte und zweckmäßige Weiterbildungen dieser Aufgabenlösung sind in den Unteransprüchen 2 bis 8 angegeben.

Vorrichtungsmäßig wird die Aufgabe gelöst durch die Ausbildung gemäß Anspruch 9. Vorteilhafte und zweckmäßige Weiterbildungen dieser Aufgabenlösung sind in den Unteransprüchen 10 bis 13 angegeben.

Die Erfindung nutzt die Erkennbarkeit spezifischer thermischer und optischer Materialeigenschaften der verschiedenen Werkstoffe zur stofflichen Identifikation dieser Werkstoffe. Die Erfindung verwendet hierfür einen lasergestützten Energieeintrag in die Werkstoffoberfläche und die Erfassung und Auswertung der örtlichen und zeitlichen IR-Wärmeabstrahlung mit Hilfe der pyrometrischen Temperaturmeßtechnik. In Abhängigkeit der thermischen und optischen Materialeigenschaften, wie Absorptionsgrad, Wärmeleitfähigkeit, Wärmekapazität und Emmissionsgrad, bilden sich sowohl zeitliche (Abkühlgeschwindigkeiten) als auch örtliche (Wärmeverteilung) stoffspezifische Unterschiede in der Wärmeimpulsantwort der Werkstoffe aus. Die Wärmeimpulsantwort ist charakterisiert durch Temperaturmaxima direkt nach der Laserbestrahlung, durch Temperaturverteilungen und Abkühlgeschwindigkeiten.

Bei einer statischen Werkstoffanalyse verwendet man zweckmäßigerweise ein Thermographiesystem (Thermokamera). Im Falle der Werkstoffanalyse von bewegten Werkstoffen kann zur Aufnahme der Wärmeimpulsantwort ein IR-Linienscanner verwendet werden, da hier die Bewegung des Werkstoffes die IR-Strahlungserfassung der Bewegungsachse automatisch ermöglicht. Die Gewinnung eines Analysesignales, das ein Sortiersignal oder ein Signal zur Erkennung von Störstellen und Inhomogenitäten im Materialgefüge sein kann, erfolgt in einer rechnergestützten Auswerteeinrichtung, die die mit Hilfe der Pyrometrie erfaßte Wärmeimpulsantwort auswertet. Hierbei können sowohl Referenzbibliotheken als auch Fuzzylogik bzw. neuronale Netze zur Auswertung der stoffspezifischen Merkmale der Wärmeimpulsantwort genutzt werden. Mit Hilfe der rechnergestützten Auswertung können bis zu 50 Analysenvorgänge pro Sekunde ausgewertet werden, so daß mit Mehrfachvermessungen einer Werkstoffprobe die Erkennungssicherheit gesteigert werden kann.

Die Verwendung eines CO₂-Lasers, dessen charakteristische Laserwellenlänge bei λ = 10,6 µm liegt, bietet aufgrund dieser Wellenlänge im IR-Bereich folgende Vorteile: Kunststoffen werden häufig Pigmentfarbstoffe zugesetzt, die in der Nähe des sichtbaren Wellenlängenbereiches die Strahlungsabsorption beeinflussen und so zu einer Störung der Wärmeimpulsantwort führen können. Im Wellenlängenbereich um 10,6 µm wirken die Farbstoffe nicht absorbierend, so daß die Wärmeimpulsantwort nicht beeinflußt wird. Durch die Verwendung der Laserstrahlung des mittleren IR-Bereichs wird der Einfluß des Oberflächenlagewinkels des zu identifizierenden Werkstoffes zum Laser minimiert. Insbesondere bei Kunststoffen tritt bei Verwendung der Laserstrahlung im mittleren IR-Bereich aufgrund der vergleichsweise hohen Absorption Oberflächenabsorption anstelle Volumenabsorption auf, so daß hier auch dünnwandige Kunststoffe untersucht werden können.

Die Erfindung soll nachfolgend anhand der beigefügten Zeichnung, in der unter anderem Ausführungsbeispiele dargestellt sind, näher erläutert werden.

Es zeigt
- Fig. 1: das Prinzip einer Vorrichtung zur lasergestützten Werkstofferkennung,
- Fig. 2: in einer grafischen Darstellung die Transmission verschiedener Werkstoffe in Abhängigkeit von der Materialstärke,
- Fig. 3: in einer grafischen Darstellung die Temperatur in Abhängigkeit von der Materialtiefe,
- Fig. 4: das Prinzip einer Vorrichtung zur lasergestützten Werkstofferkennung bei bewegten Werkstoffen,
- Fig. 5: eine Frontansicht einer bevorzugten Ausführungsform der Vorrichtung nach Fig. 4,
- Fig. 6: eine Seitenansicht der Vorrichtung nach Fig. 5,
- Fig. 7: schematisch eine Vorrichtung zur Erfassung der Abkühlgeschwindigkeit bei bewegten Werkstoffen,
- Fig. 8: das Prinzip einer Vorrichtung zur lasergestützten Werkstofferkennung bei ruhenden Werkstoffen,
- Fig. 9: eine Tabelle bestimmter Stoffeigenschaften verschiedener Kunststoffe,
- Fig. 10: in einer grafischen Darstellung das Temperaturzeitverhalten von fünf verschiedenen Kunststoffen,
- Fig. 11 und 12: in einer grafischen Darstellung die Durchschnitts- und Maximaltemperaturen sowie die Temperaturverteilungen verschiedener Kunststoffe auf den mit Laserlicht bestrahlten Oberflächen,
- Fig. 13: schematisch eine Vorrichtung zur Einstrahlung mehrerer Laserimpulse verschiedener Energie und
- Fig. 14: schematisch eine Vorrichtung bei Verwendung eines Lasers im Dauerstrichbetrieb und verschiedener Strahlenergien.

Soweit sinnvoll, werden in den Figuren der Zeichnung für gleiche Teile die gleichen Bezugszeichen verwendet.

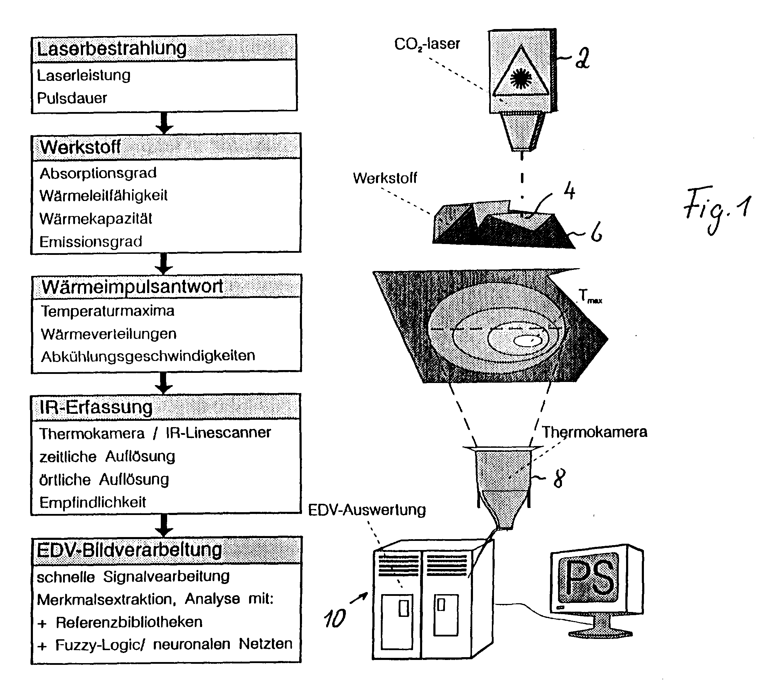

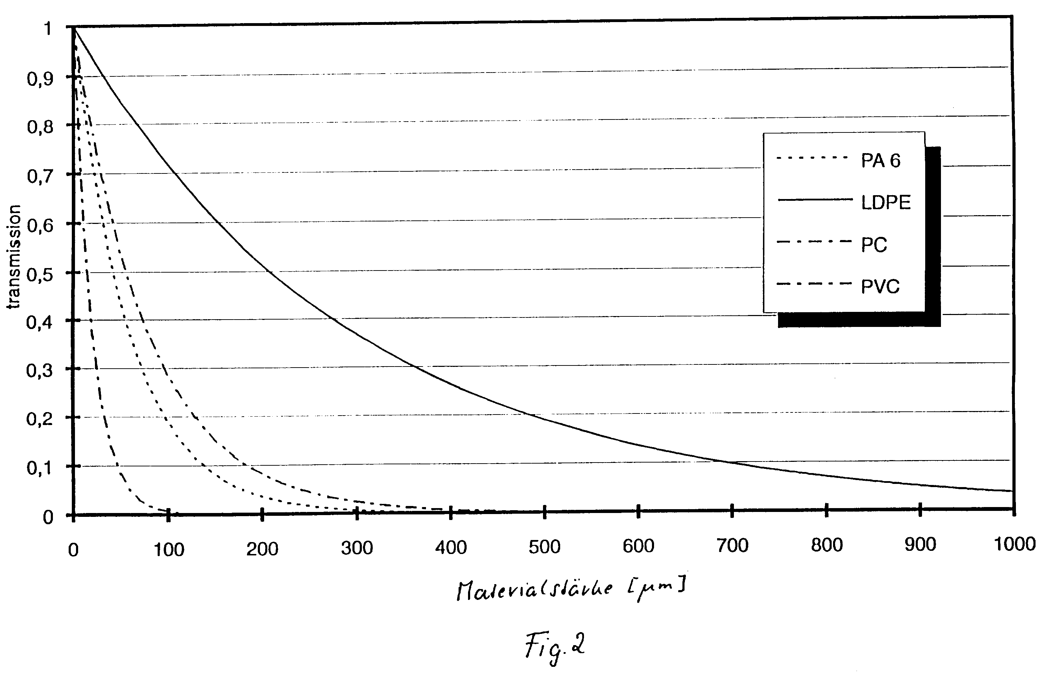

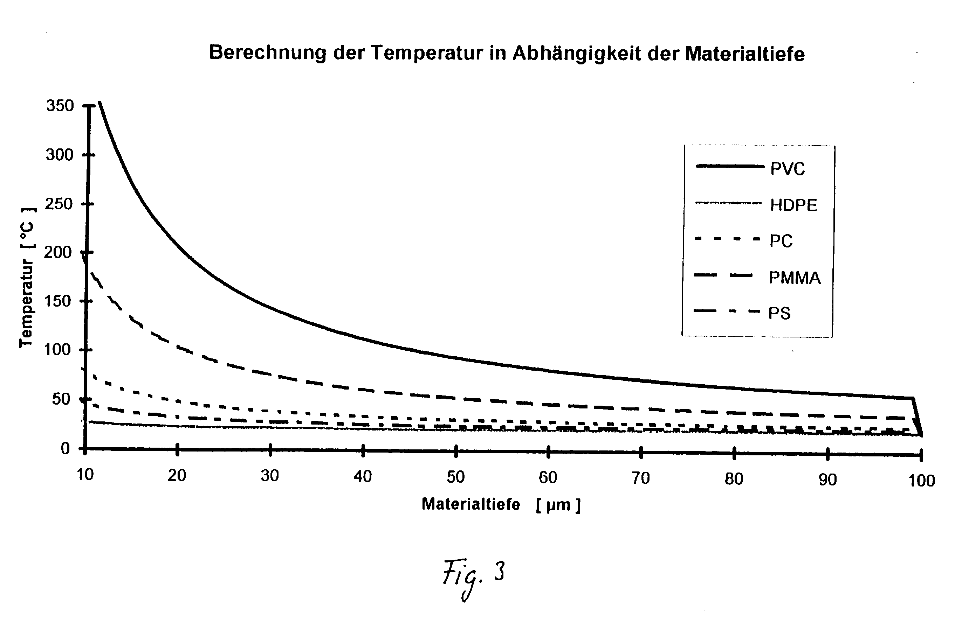

Die Fig. 1 zeigt schematisch eine Vorrichtung zur lasergestützten Werkstofferkennung. Mit Hilfe eines CO₂-Lasers 2 wird eine Stelle 4 eines zu identifizierenden Werkstoffes 6 mit Laserenergie beaufschlagt, und zwar punktweise und kurzzeitig. Es können Laserbestrahlungen mit Leistungen unter 300 Watt und mit einer Pulsdauer unter 20 ms eingesetzt werden. Mit Hilfe einer Thermokamera 8 wird dann sofort nach der Energiebeaufschlagung die Wärmeimpulsantwort des Werkstoffes erfaßt. Bei der Analyse von bewegten Werkstoffen wird vorzugsweise ein IR-Linienscanner verwendet. Unterschiedliche Werkstoffe lassen sich hinsichtlich ihrer thermischen und optischen Materialeigenschaften, wie Absorptionsgrad, Wärmeleitfähigkeit, Wärmekapazität und Emissionsgrad, untersuchen. In Abhängigkeit dieser Materialeigenschaften ergeben sich hinsichtlich Abkühlgeschwindigkeiten, Wärmeverteilungen und Temperaturmaxima stoffspezifische Unterschiede in der Wärmeimpulsantwort der bestrahlten Werkstoffe. Insbesondere bei Kunststoffen ist das Absorptionsverhalten der verschiedenen Kunststoffsorten stark unterschiedlich (vgl. Fig. 2 und 3), woraus u. a. die Ausbildung unterschiedlicher maximaler Temperaturen resultiert, die besonders gut auswertbar sind.

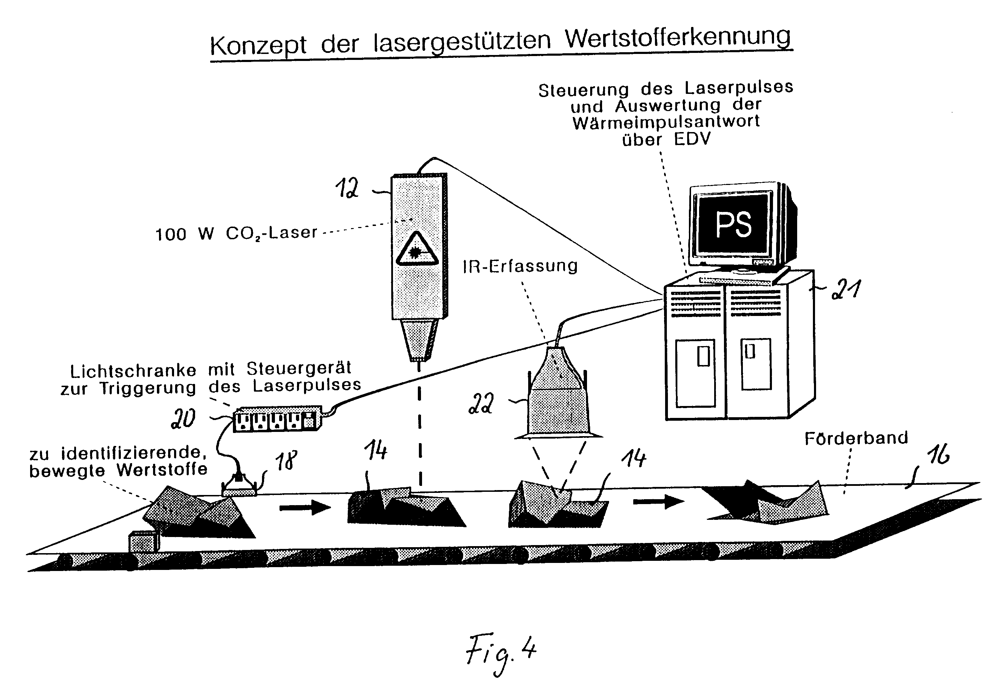

Die Fig. 4 bis 7 zeigen eine Vorrichtung zur lasergestützten Werkstofferkennung bei bewegten Werkstoffen. Ein CO₂-Laser 12 mit beispielsweise einer Leistung von 100 Watt beaufschlagt zu identifizierende Werkstoffe 14, die mittels Förderband 16 durch eine Prüfstrecke transportiert werden. Der Eintritt der Werkstoffe in die Prüfstrecke wird von einem Sensor 18, beispielsweise einer Lichtschranke, sensiert, dessen Ausgangssignal ein Steuergerät 21 zur Triggerung des Laserpulses zugeführt wird, das den Laser 12 zur Bestrahlung der den Sensor passierenden Werkstoffe in Abhängigkeit vom Sensorsignal über eine elektronische Datenverarbeitungsanlage 20 steuert. Mit Hilfe eines IR-Linienscanners 22 wird die Wärmeimpulsantwort aufgenommen und zur Auswertung der elektronischen Datenverarbeitungsanlage 21 zugeführt.

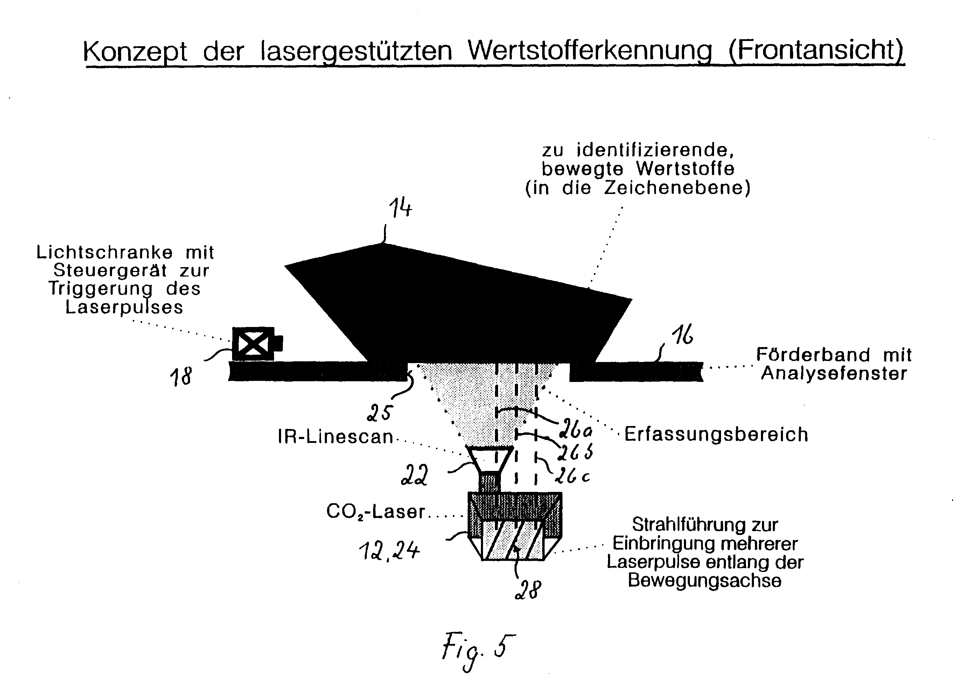

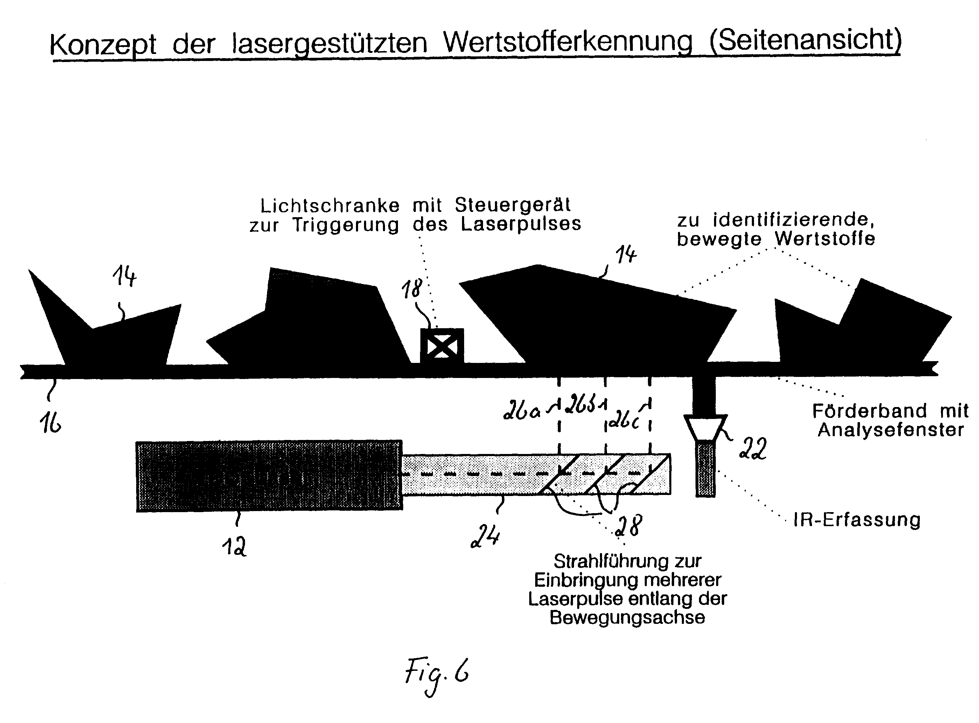

Bevorzugt ist der Laser 12 mit seiner Optik 24 unterhalb der zu identifizierenden Werkstoffe angeordnet, wie dies in den Fig. 5 und 6 dargestellt ist, um die Werkstoffoberfläche von unten mit Strahlungsenergie zu beaufschlagen. Hierzu weist das Förderband 16 ein Analysefenster 25 auf, über das die Energieeinstrahlung und die Erfassung der Wärmeimpulsantwort erfolgt. Dies hat den Vorteil, daß unterschiedliche Abstände des Werkstoffes zum Analysesystem, die durch variierende Werkstoffgrößen verursacht werden, minimiert werden. Ein weiterer Vorteil ist die Minimierung der Variation der Oberflächenlagewinkel zum Analysesystem.

Ferner kann die Werkstoffoberfläche zeitgleich mit mehreren Laserpulsen 26a, 26b, 26c beaufschlagt werden unter Verwendung eines Mehrfachstrahlteilers 28, wie dies in Fig. 5, 6 und 7 schematisch dargestellt ist. Hierzu ist eine Mehrfachvermessung und eine Steigerung der Erkennungssicherheit (auch von ruhenden Werkstoffen) möglich. Ferner ist es hierdurch möglich, Abkühlgeschwindigkeiten von bewegten Werkstoffproben zu messen, wenn die gleichzeitige Beaufschlagung mit mehreren Laserpulsen entlang der Bewegungsachse erfolgt.

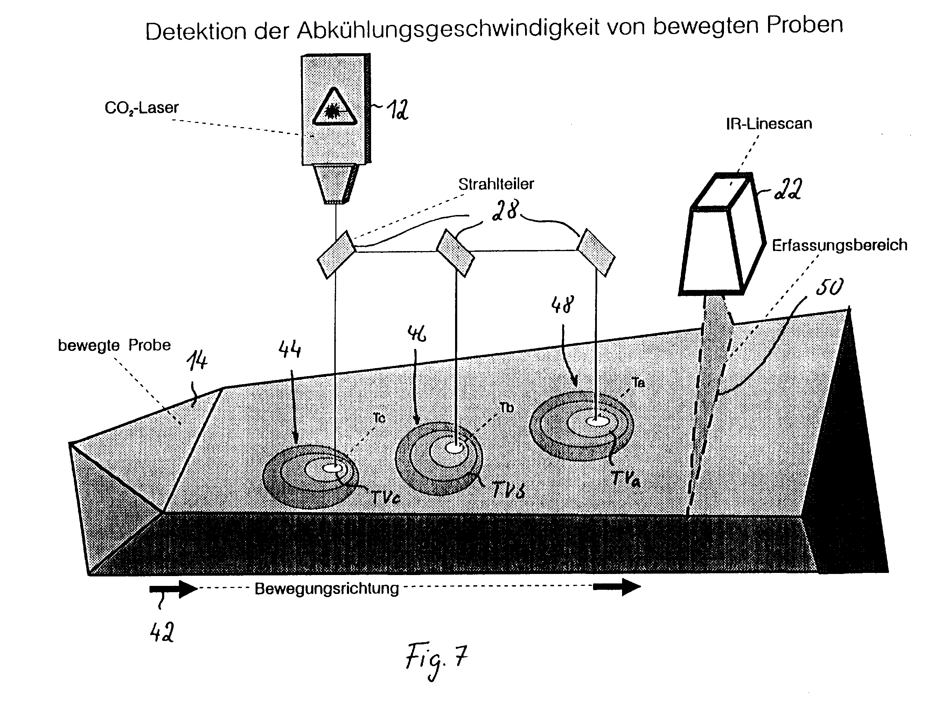

Die Fig. 7 zeigt speziell eine Anordnung zur Erfassung der Abkühlgeschwindigkeit von bewegten Werkstoffen 14, indem beispielsweise wie dargestellt, drei in Bewegungsrichtung 42 hintereinander liegende Oberflächenbereiche 44, 46 und 48 gleichzeitig, punktförmig und kurzzeitig mit Hilfe des Lasers 12 und des Mehrfachstrahlteilers 28 bestrahlt werden und anschließend zeitlich nacheinander den Erfassungsbereich 50 des IR-Linienscanners 22 passieren. Der IR-Linienscanner 22 kann dabei sowohl die Abnahme der Maximaltemperaturen Ta, Tb und Tc als auch die zugeordneten Temperaturverteilungen, die durch die Isothermen TVa, TVb und TVc angedeutet sind, erfassen.

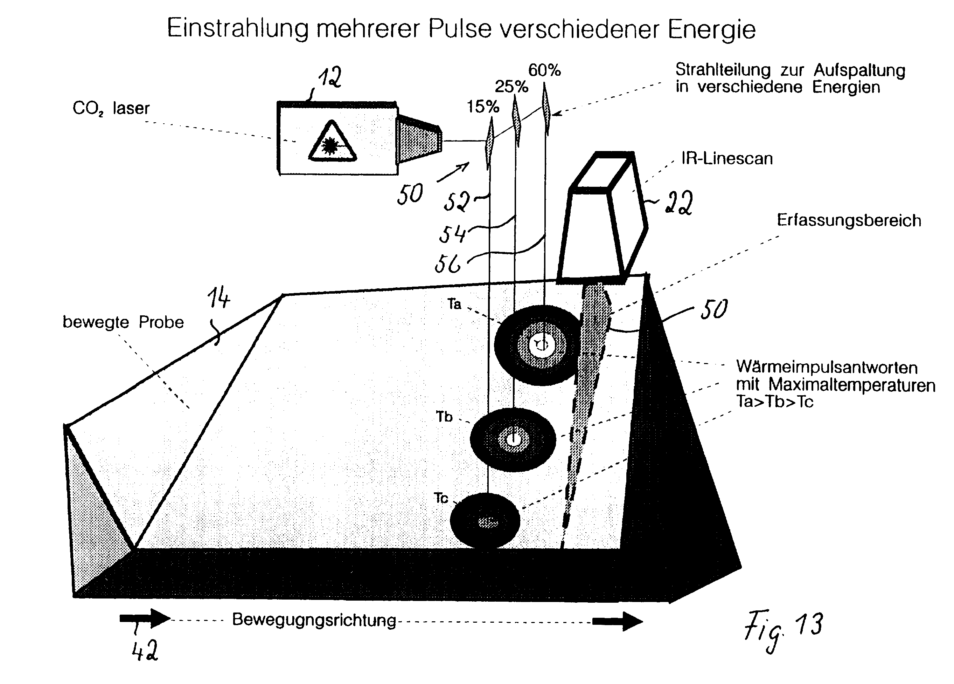

Die Fig. 13 zeigt eine Anordnung mit einem Mehrfachstrahlteiler 50, mit dem es möglich ist, zeitgleich mehrere Laserpulse 52, 54, 56 unterschiedlicher Laserenergie in die Werkstoffoberfläche (z. B. senkrecht zur Bewegungsachse) einzubringen.

Die gleichzeitige Vermessung der unterschiedlichen resultierenden Wärmeimpulsantworten hat den Vorteil der Aufweitung der meßbaren Unterschiede zwischen den verschiedenen zu identifizierenden Werkstoffen (insbesondere bei Kunststoffen bei evtl. vorhandenen ähnlichen Wärmeimpulsantworten der verschiedenen Kunststoffe).
1. Bei schwach absorbierenden Kunststoffen wie z. B. PE und PP ist zur Erzeugung optimaler Unterschiede (grossen Temperaturdifferenzen) der Wärmeimpulsantworten die Verwendung von relativ hohen Laserenergien zu empfehlen (z. B. bei 1-2mm Strahldurchmesser, 70W 20ms oder 1,4 J).
2. Bei stark absorbierenden Kunststoffen führt demgegenüber eine zu hohe Laserenergie zu Temperaturen >250°C (nahe der Schmelztemperatur), mit dem Nachteil, daß unter Einwirkung der erzeugten Temperaturen teilweise Schmelzen und Verdampfen eintritt, was durch die aufzubringende Schmelz- und Verdampfungsenergie zu geringeren und wenig reproduzierbaren Temperaturverteilungen (und maximalen Temperaturen) führt. Die aufzuwendende Schmelz- und Verdampfungswärme begrenzt somit erreichbare maximale Temperaturen auf ca. 340°C (ohne Flammenbildung). Zur Unterscheidung stark absorbierender Kunststoffe wie PC, PMMA oder PVC sind somit niedrigere Laserenergien (ca. 0,8J) von Vorteil.

Bei gleichzeitiger Verwendung mehrerer Laserpulse unterschiedlicher Energie kann man somit die Auswirkungen der stoffspezifischen Absorptionskoeffizienten verstärken und somit eine größere Anzahl von verschiedenen Kunststoffen in einem Meßvorgang analysieren und zusätzlich die Erkennungssicherheit steigern.

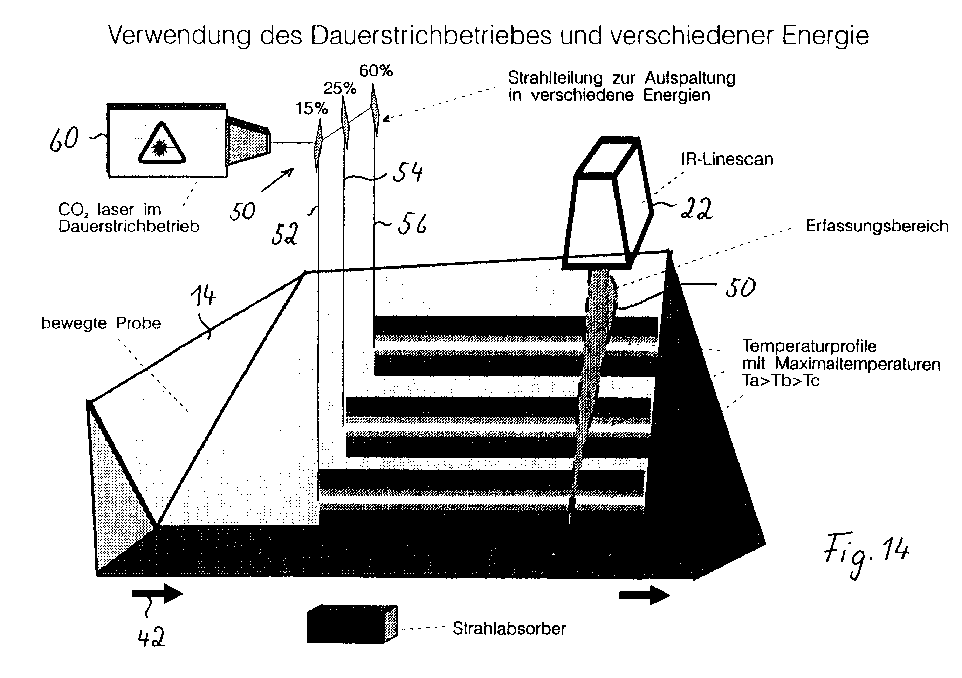

Bei der bewegten Werkstofferkennung kann ein Laser 60 im Dauerstrichbetrieb (gepulst oder ungepulst) mit einem Mehrfachstrahlteiler 50 zur zeitgleichen Einbringung mehrerer Laserstrahlen unterschiedlicher Energie (analog wie bei der Vorrichtung nach der Fig. 13) von Vorteil sein, vgl. Fig. 14. Vorteile sind insbesondere die nicht notwendige Triggerung des Lasers, da die bewegten Werkstoffproben in jedem Fall vom Laser erfaßt werden. Ein weiterer Vorteil ist die Erhöhung der Anzahl der auswertbaren Temperaturprofile, da auf der gesamten bestrahlten Werkstofflänge auswertbare IR-Wärmestrahlung zu beobachten ist. Nennenswerte Nachteile der genannten Anordnung ist die Unmöglichkeit mit Hilfe eines Linien- bzw. Zeilenscanners das Abkühlungsverhalten zu detektieren und die Notwendigkeit einer höheren Laserenergieaufnahme sowie die damit verbundene Verkürzung der mittleren Lebensdauer des eingesetzten Lasers.

Zur Vermeidung von Schädigungen des bestrahlten Untergrundes, wenn sich kein zu analysierendes Werkstück im Strahlengang befindet, umfaßt die Anordnung in diesem Fall einen Strahlabsorber (mit guter Wärmeleitfähigkeit und hoher thermischer Beständigkeit).

Die gleichzeitige Verwendung mehrerer Laser verschiedener Wellenlängen bietet den Vorteil der möglichen Berücksichtigung verschiedener wellenlängenabhängiger Absorptionskoeffizienten und ermöglicht dadurch eine Erhöhung der Erkennungssicherheit. Zu berücksichtigen ist hierbei der höhere apparative Aufwand der Anordnung.

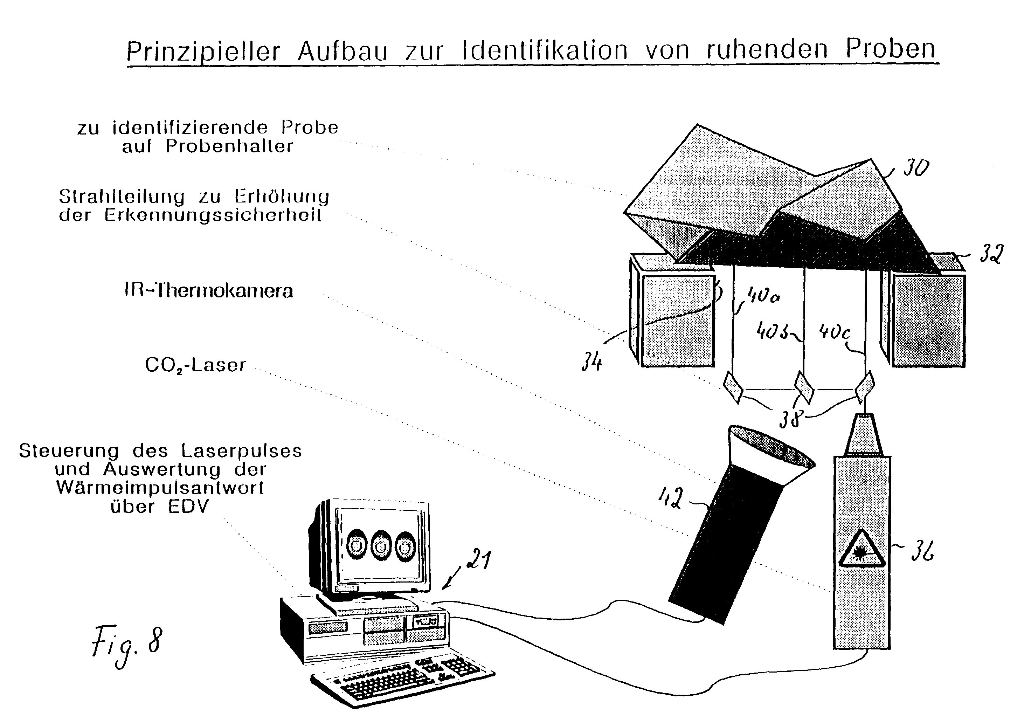

Die Fig. 8 zeigt eine Vorrichtung zur lasergestützten Werkstofferkennung bei ruhenden Werkstoffen. Der zu identifizierende Werkstoff 30 ruht auf einer Unterlage 32, die ein Analysefenster 34 aufweist. Ein CO₂-Laser 36 beaufschlagt die Werkstoffoberfläche über das Analysefenster 34 von unten mit Strahlungsenergie, wobei auch hier, wie bei der Ausführungsform nach den Fig. 5 und 6, der Laserstrahl durch einen Mehrfachstrahlaufspalter 38 in mehrere Laserstrahlen 40a, 40b, 40c aufgeteilt wird, um den Werkstoff mit mehreren Laserpulsen gleichzeitig, punktweise und kurzzeitig zu beaufschlagen zum Zweck der Mehrfachvermessung und damit zur Steigerung der Erkennungssicherheit. Mit Hilfe einer Thermokamera 42 wird die Wärmeimpulsantwort der Werkstoffe erfaßt. Es können auch Laserpulse unterschiedlicher Energie eingebracht werden wie bei den Ausführungsformen nach den oben beschriebenen Figuren 13 und 14.

Die Auswertung der Temperaturmaxima, Tempraturverteilungen und Abkühlgeschwindigkeiten aus der Wärmeimpulsantwort der pyrometrischen Temperaturmeßvorrichtungen und die Gewinnung des Analysesignales (beispielsweise eines Sortiersignales) erfolgt in den rechnergestützen Auswerteeinrichtungen 10 und 21, vgl. Fig. 1, 4 und 8. Hierbei werden sowohl Referenzbibliotheken als auch eine Fuzzylogik bzw. neuronale Netze zur Auswertung der stoffspezifischen Merkmale der Wärmeimpulsantwort genutzt.

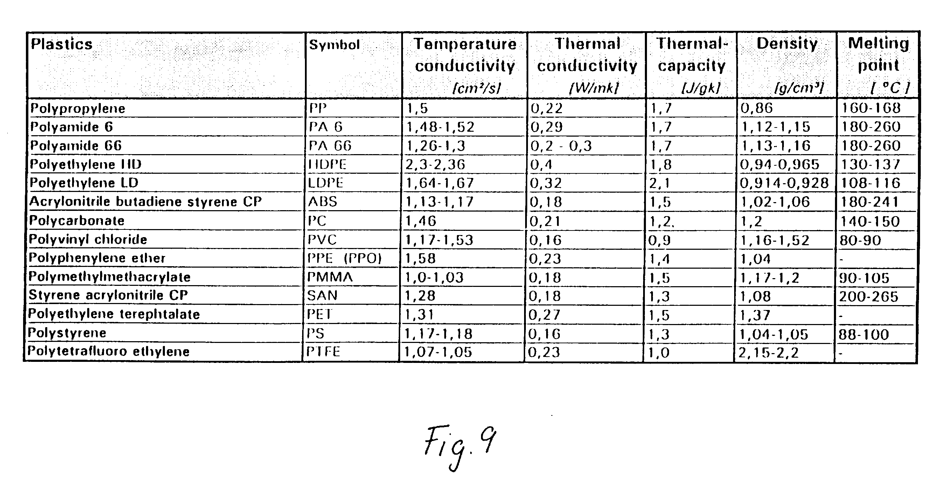

Die Fig. 9 zeigt eine Tabelle bestimmter Stoffeigenschaften verschiedener Kunststoffe und bedarf keiner weiteren Erläuterung.

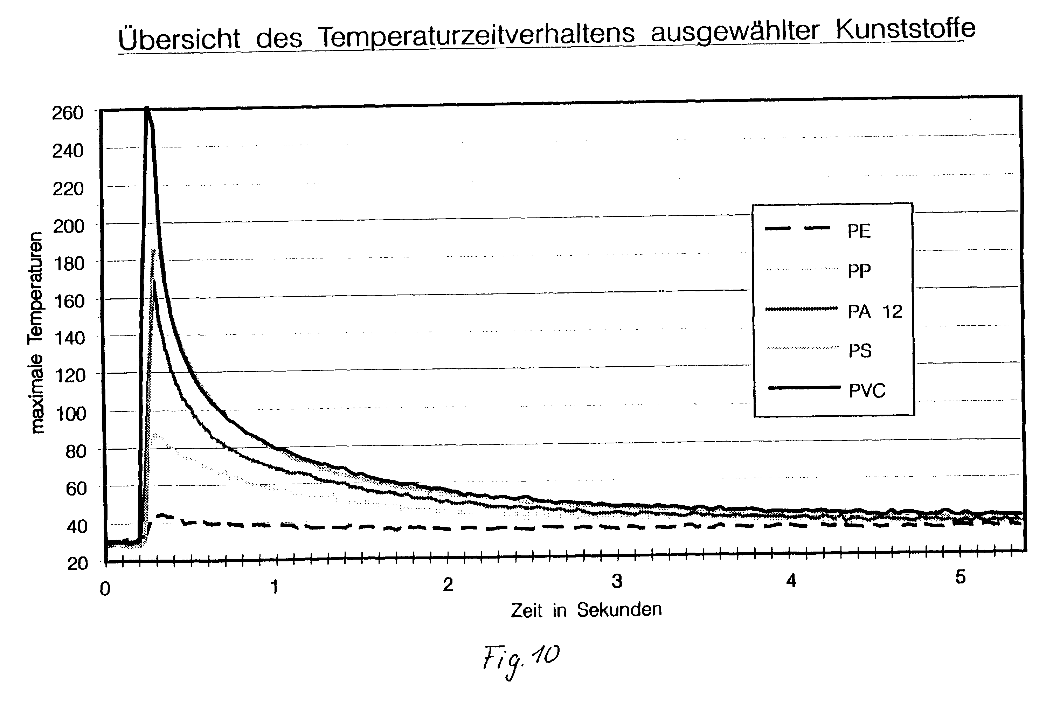

Die Fig. 10 zeigt das Temperaturzeitverhalten auf der Oberfläche bestrahlter Kunststoffe, nämlich PVC, PS, PA 12, PP und PE, wobei unter Verwendung eines Lasers mit beispielsweise einer Leistung von 60 Watt die Oberflächen der Kunststoffe innerhalb einer Zeit von 1/10 Sekunde auf bis zu 262°C erwärmt wurden. Die Temperaturkurven zeigen sehr deutlich die materialspezifischen Unterschiede der untersuchten Kunststoffe. Der geringste, aber noch deutlich erkennbare Unterschied besteht zwischen PP und PA 12.

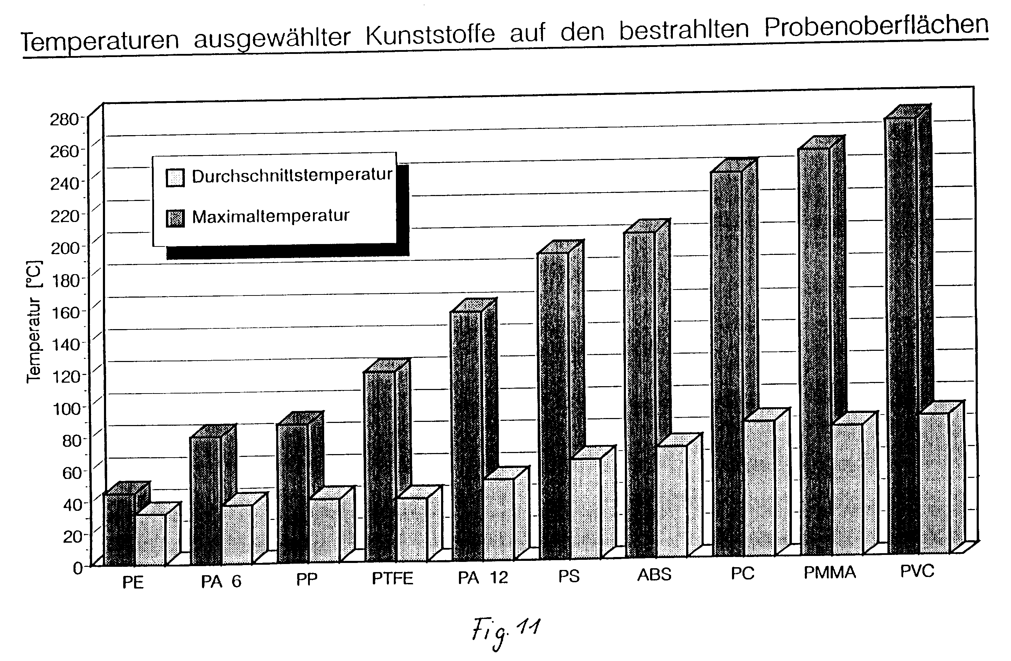

Die Fig. 11 zeigt die Maximaltemperatur und Durchschnittstemperatur im bestrahlten Oberflächenausschnitt direkt nach der Energieeinkopplung bei den Kunststoffen PE, PA 6, PP, PTFE, PA 12, PS, ABS, PC, PMMA, PVC. Die Maximaltemperaturen und die mittleren Temperaturen wurden direkt nach der Bestrahlung innerhalb des bestrahlten Werkstückoberflächenbereiches gemessen. Auffällig sind die sehr großen Unterschiede bei den Kunststoffen PE, PP, PS, ABS und PVC, die sowohl im Hausmüll als auch im Bereich der Elektroschrottverwertung vermehrt anfallen. Die geringsten, dennoch auswertbaren Temperaturunterschiede von nur 7°C treten zwischen PP und PA 6 auf. Ein anderes, aber ebenfalls werkstoffspezifisches Resultat ergibt sich durch die Auswertung anderer IR-Erfassungsgrößen, wie z. B. der Flächenverteilung innerhalb der bestrahlten Werkstückoberfläche oder des Temperaturzeitverhaltens.

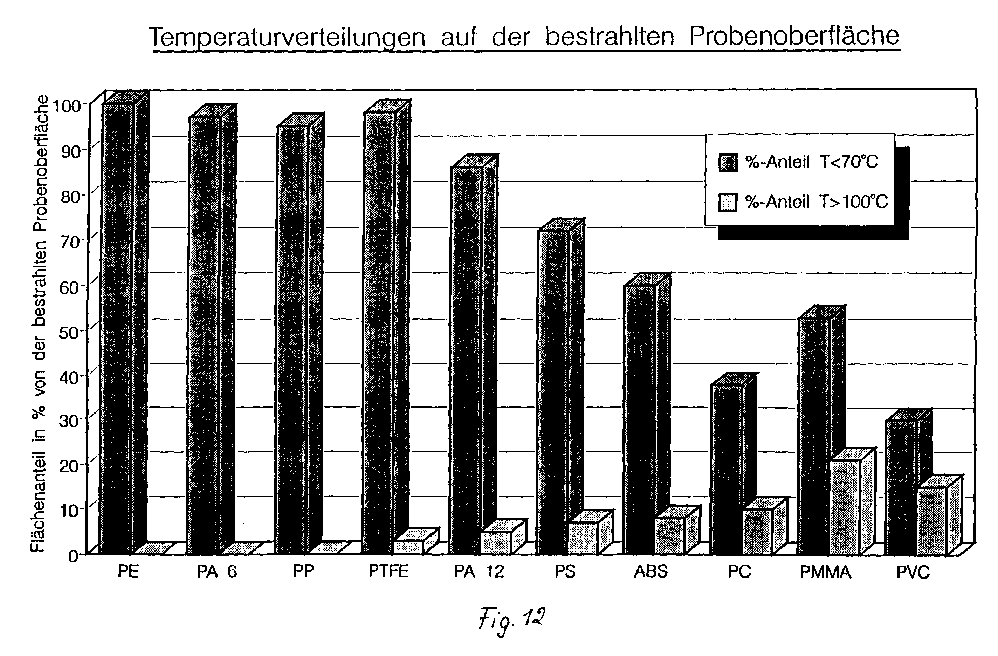

Die Fig. 12 zeigt für die oben im Zusammenhang mit Fig. 11 bereits genannten Kunststoffe die Temperaturverteilung auf dem bestrahlten Oberflächenbereich direkt nach der Bestrahlung der Kunststoffe. Betrachtet man den Verlauf der Temperaturflächenanteile in Prozent von der bestrahlten Werkstoffoberfläche, so wird deutlich, daß erwartungsgemäß ein Anstieg der Flächenanteile hoher Temperatur (T>100°C) und ein Abfall der Flächenanteile mit T<70°C zu beobachten ist. Dennoch zeigen sich bei Berücksichtigung unterschiedlicher Auswertemethoden wichtige Unterschiede, die zur Erhöhung der Erkennungssicherheit beitragen können. Beim Vergleich der Fig. 2 und 3 wird deutlich, daß in Abhängigkeit des verwendeten Auswerteverfahrens besimmte stoffspezifische Unterschiede hervorgehoben werden können. Beispielsweise lassen sich PC, PMMA und PVC, die bei der Auswertung nach Fig. 2 relativ schwer unterscheidbar sind, bei der Auswertung der Temperaturflächenanteile nach Fig. 3 viel deutlicher unterscheiden.

Die wie oben beschrieben durchgeführten Messungen ergaben folgende Ergebnisse:
1. Aufgrund der kurzen Einwirkdauer der Laserstrahlung konnte am untersuchten Kunststoff nur eine zu vernachlässigende Schädigung der Werkstoffoberfläche nachgewiesen werden.
2. Unterschiedliche Werkstoffdicken, Werkstofformen sowie Werkstoffoberflächen wirkten sich nicht störend bei der Erkennung der Werkstoffe aus.
3. Der Einfluß von Einfärbungen und unterschiedlichen Additivzusätzen (Weichmachern) erwies sich bei den untersuchten Kunststoffen ebenfalls als nicht störend.
4. Bei allen untersuchten Kunststoffen PE, PA 6, PP, PTFE, PA 12, PS, ABS, PC, PMMA und PVC konnte bereits aufgrund der Auswertung der Temperaturen zum Zeitpunkt der maximalen Aufheizung des Kunststoffes, somit direkt nach der Laserbestrahlung, eine eindeutige Identifikation des Werkstoffes herbeigeführt werden. Eine Analyse der Werkstoffe ist innerhalb 1/100 Sekunde möglich.
5. Das beschriebene Verfahren zur Werkstofferkennung ist nicht nur für Kunststoffe anwendbar, sondern auch für alle anderen Werkstoffe, beispielsweise Glas, Metalle etc., die sich bzgl. der oben genannten Materialeigenschaften unterscheiden.
6. Es ist möglich, Werkstoffe innerhalb anderer Werkstoffgruppen zu identifizieren.

## Patentansprüche

1. Verfahren zur stofflichen Identifikation von Werkstoffen, bei dem die Werkstoffe mit Laserenergie beaufschlagt werden und die Wärmeabstrahlung der Werkstoffe nach Beendigung der Erwärmung erfaßt und werkstoffspezifisch hinsichtlich optischer und thermischer Materialeigenschaften ausgewertet wird, **dadurch gekennzeichnet,** daß wenigstens eine Stelle der Oberfläche der zu identifizierenden Werkstoffe punktförmig und kurzzeitig mit der Laserenergie beaufschlagt wird und die für den beaufschlagten Oberflächenbereich stofflich charakteristische Wärmeimpulsantwort des Werkstoffes örtlich und/oder zeitlich meßtechnisch erfaßt und hinsichtlich der Werkstoffart ausgewertet wird, indem die Maximaltemperatur, die ein Maß für die wellenlängenabhängige Absorption bzw. optische Eindringtiefe des Werkstoffes darstellt, direkt nach der lasergestützten Erwärmung und/oder die Wärmeverteilung, die ein Maß für die Strahlungsabsorption und für die Energieverteilung des Laserstrahles auf der Werkstoffoberfläche ist, direkt nach der Laserbestrahlung und/oder die Wärmeverteilung nach einer definierten Abkühlzeit nach Beendigung der Laserbestrahlung als ein Maß für die Temperaturleitfähigkeit des zu identifizierenden Werkstoffes unter Berücksichtigung der eingebrachten Temperaturverteilung direkt nach der Laserbestrahlung und/oder die Abkühlgeschwindigkeiten, die bei Berücksichtigung der direkt nach der Laserstrahlung erzeugten Temperaturgradienten im Material des Werkstoffes ein Maß für die Temperaturleitfähigkeit darstellen, gemessen werden, wobei in jedem Fall die Wärmeimpulsantwort durch eine pyrometrische Temperaturmessung mit Hilfe einer Thermokamera oder eines IR-Linien- bzw. Zeilenscanners erfaßt wird.

2. Verfahren zur stofflichen Identifikation von Werkstoffen, bei dem die Werkstoffe mit Laserenergie beaufschlagt werden und die Wärmeabstrahlung der Werkstoffe nach Beendigung der Erwärmung erfaßt und werkstoffspezifisch hinsichtlich optischer und thermischer Materialeigenschaften ausgewertet wird, **dadurch gekennzeichnet,** daß wenigstens eine Stelle der Oberfläche der zu identifizierenden Werkstoffe punktförmig und kurzzeitig mit der Laserenergie beaufschlagt wird und die für den beaufschlagten Oberflächenbereich stofflich charakteristische Wärmeimpulsantwort des Werkstoffes örtlich und/oder zeitlich meßtechnisch erfaßt und hinsichtlich der Werkstoffart ausgewertet wird, indem die Maximaltemperatur, die ein Maß für die wellenlängenabhängige Absorption bzw. optische Eindringtiefe des Werkstoffes darstellt, direkt nach der lasergestützten Erwärmung und/oder die Wärmeverteilung, die ein Maß für die Strahlungsabsorption und für die Energieverteilung des Laserstrahles auf der Werkstoffoberfläche ist, direkt nach der Laserbestrahlung und/oder die Wärmeverteilung nach einer definierten Abkühlzeit nach Beendigung der Laserbestrahlung als ein Maß für die Temperaturleitfähigkeit des zu identifizierenden Werkstoffes unter Berücksichtigung der eingebrachten Temperaturverteilung direkt nach der Laserbestrahlung und/oder die Abkühlgeschwindigkeiten, die bei Berücksichtigung der direkt nach der Laserstrahlung erzeugten Temperaturgradienten im Material des Werkstoffes ein Maß für die Temperaturleitfähigkeit darstellen, gemessen werden, und
daß in jedem Fall zur Identifikation von bewegten Werkstoffen in einem automatischen Prozeß der Laser durch ein vom Eintritt der zu analysierenden Werkstoffe in eine Prüfstrecke abhängiges Triggersignal gesteuert wird.

3. Verfahren nach einem der vorhergehenden Ansprüche, **dadurch gekennzeichnet,** daß die Werkstoffoberfläche zeitgleich mit mehreren Laserpulsen beaufschlagt wird.

4. Verfahren nach einem der vorhergehenden Ansprüche, **dadurch gekennzeichnet,** daß der Werkstoff mit mehreren Laserstrahlen unterschiedlicher Intensität beaufschlagt wird.

5. Verfahren nach einem der vorhergehenden Ansprüche, **dadurch gekennzeichnet,** daß der Werkstoff mit mehreren Laserstrahlen unterschiedlicher Wellenlänge beaufschlagt wird.

6. Verfahren nach einem der vorhergehenden Ansprüche, **dadurch gekennzeichnet,** daß der Werkstoff während seiner Bewegung durch den Wirkbereich des Lasers entlang seiner Bewegungsachse gleichförmig mit der Laserstrahlung beaufschlagt wird.

7. Verfahren nach einem der vorhergehenden Ansprüche, **dadurch gekennzeichnet,** daß zusätzlich der Temperaturgradient der Wärmeimpulsantwort gemessen wird und hinsichtlich des Oberflächenlagewinkels des Werkstoffes zum Laser ausgewertet wird zur entprechenden Korrektur der Wärmeimpulsantwort.

8. Verfahren nach einem der vorhergehenden Ansprüche, **dadurch gekennzeichnet**, daß die Dauer der Beaufschlagung der Werkstoffe mit Laserenergie 5-50 msec beträgt.

9. Verfahren nach einem der vorhergehenden Ansprüche, **dadurch gekennzeichnet,** daß ein Strahldurchmesser ≤ 2mm verwendet wird.

10. Verfahren nach einem der vorhergehenden Ansprüche, **dadurch gekennzeichnet,** daß ein oder mehrere Einzelstrahlen mit jeweils einer Leistung ≤ 100 Watt verwendet werden.

11. Vorrichtung zur Durchführung des Verfahrens nach einem der vorhergehenden Ansprüche, bei ruhendem zu identifizierenden Werkstoff mit einem Laser zur kurzzeitigen Beaufschlagung wenigstens einer Stelle der Oberfläche des zu identifizierenden Werkstoffes, einer Temperaturmeßeinrichtung und einer an die Temperaturmeßeinrichtung angeschlossenen rechnergestützten Auswerteeinrichtung, **dadurch gekennzeichnet,** daß für die Erfassung der Wärmeimpulsantwort des zu identifizierenden Werkstoffes (6, 14, 30) eine Thermokamera (8, 42) oder ein IR-Linienscanner (22) vorgesehen ist, deren aus der pyrometrischen Temperaturmessung gewonnenen Meßsignale der rechnergestützten Auswerteeinrichtung (10, 21) zugeführt werden, welche die Meßsignale gemäß einem der vorhergehenden Ansprüche hinsichtlich Temperaturmaxima und/oder Temperaturverteilungen und/oder Abkühlgeschwindigkeiten auswertet und Analysesignale bereitstellt unter Nutzung von Referenzbibliotheken oder Fuzzylogiken oder neuronaler Netze.

12. Vorrichtung zur Durchführung des Verfahrens nach einem der Ansprüche 1 bis 10 bei bewegten zu identifizierenden Werkstoffen mit einem Laser zur kurzzeitigen Beaufschlagung wenigstens einer Stelle der Oberfläche des zu identifizierenden Werkstoffes, einer Transporteinrichtung für die zu untersuchenden Werkstoffe, einer Temperaturmeßeinrichtung und einer an die Temperaturmeßeinrichtung angeschlossenen rechnergestützen Auswerteeinrichtung, **dadurch gekennzeichnet,**
daß die aus der pyrometrischen Temperaturmessung gewonnenen Meßsignale der rechnergestützten Auswerteeinrichtung (10, 21) zugeführt werden, welche die Meßsignale gemäß einem der Ansprüche 1 bis 10 hinsichtlich Temperaturmaxima und/oder Temperaturverteilungen und/oder Abkühlgeschwindigkeiten auswertet und Analysesignale bereitstellt unter Nutzung von Referenzbibliotheken oder Fuzzylogiken oder neuronaler Netze, wobei ein Sensor (18) vorgesehen ist, der den Eintritt eines Werkstoffes (14) in eine Prüfstrecke erfaßt, sowie ein Triggersignalgeber (20), der vom Sensorausgangssignal gesteuert wird und mit dem Laser (12) verbunden ist zu dessen Steuerung in Abhängigkeit vom Sensorausgangssignal.

13. Vorrichtung nach Anspruch 12, **dadurch gekennzeichnet,** daß ein Laser (60) im Dauerstrichbetrieb verwendet wird.

14. Vorrichtung nach Anspruch 12 oder 13, **dadurch gekennzeichnet,** daß entlang der Bewegungsrichtung oder quer zur Bewegungsrichtung des Werkstoffes mehrere Laser oder ein Mehrfachstrahlteiler (28, 38, 50) für die Laserstrahlung angeordnet sind bzw. ist.

15. Vorrichtung nach Anspruch 14, **dadurch gekennzeichnet,** daß ein Mehrfachstrahler (50) zur Erzeugung von Laserstrahlen verschiedener Intensität verwendet wird.

16. Vorrichtung nach einem der Ansprüche 11 bis 15, **dadurch gekennzeichnet,** daß die Laserlichtoptik (12, 24, 28, 36, 38) unterhalb des Werkstoffes (6, 14, 30) angeordnet ist zur Beaufschlagung der Oberfläche des Werkstoffes von unten.

17. Vorrichtung nach einem der Ansprüche 11 bis 16, **dadurch gekennzeichnet,** daß als Laser ein CO₂-Laser eingesetzt wird.

18. Vorrichtung nach einem der Ansprüche 11 bis 17, **dadurch gekennzeichnet,** daß mehrere Laser mit verschiedenen Wellenlängen verwendet werden.

## Claims

1. A method of material identification of raw materials in which the
raw materials are subjected to laser power and after termination of the heating the radiation of heat from the raw materials is detected and evaluated as regards the optical and thermal properties of the material specifically to the raw material, characterized in that
at least one place on the surface of the material to be identified is subjected briefly to the laser power in one spot and the heat impulse reply from the raw material which is materially characteristic of the part of the surface being acted upon is detected according to place and/or time by measurement technology and evaluated as regards the kind of raw material, by measuring the maximum temperature, which represents a measure of the wavelength-dependent absorption or optical depth of penetration of the raw material, directly after the laser-dependent heating, and/or the distribution of heat after a defined cooling time after termination of the laser radiation, as a measure of the temperature conductivity of the raw material to be identified, whilst taking into consideration the temperature distribution introduced directly after the laser radiation, and/or the rates of cooling which in taking into consideration the temperature gradients generated in the material of the raw material directly after the laser radiation represent a measure of the temperature conductivity, the heat impulse reply being in any case detected by a pyrometric temperature measurement with the aid of a thermocamera or an infrared line or row scanner.

2. A method of material identification of raw materials in which the
raw materials are subjected to laser power and after termination of the heating the radiation of heat from the raw materials is detected and evaluated as regards the optical and thermal properties of the material specifically to the raw material, characterized in that
at least one place on the surface of the material to be identified is sub jected briefly to the laser power in one spot and the heat impulse reply from the raw material which is materially characteristic of the part of the surface being acted upon is detected according to place and/or time by measurement technology and evaluated as regards the kind of raw material, by measuring the maximum temperature, which represents a measure of the wavelength-dependent absorption or optical depth of penetration of the raw material, directly after the laser-dependent heating, and/or the distribution of heat after a defined cooling time from termination of the laser radiation, as a measure of the temperature conductivity of the raw material to be identified, whilst taking into consideration the temperature distribution introduced directly after the laser radiation, and/or the rates of cooling which in taking into consideration the temperature gradients generated in the material of the raw material directly after the laser radiation represent a measure of the temperature conductivity, and that
in any case for the identification of raw materials moving in an automatic process the laser is controlled by a trigger signal dependent upon the entry into a test section, of the raw materials to be analysed.

3. A method as in one of the preceding Claims, characterized in that
the surface of the raw material is subjected at equal intervals to a number of laser pulses.

4. A method as in one of the preceding Claims, characterized in that
the raw material is subjected to a number of laser beams of different intensities.

5. A method as in one of the preceding Claims, characterized in that
the raw material is subjected to a number of laser beams of different wavelengths.

6. A method as in one of the preceding Claims, characterized in that
during the motion of the raw material through the active range of the laser it is subjected uniformly to the laser radiation along its axis of motion.

7. A method as in one of the preceding Claims, characterized in that
in addition the temperature gradient of the heat impulse reply is measured and evaluated with regard to the angular position of the surface of the raw material with respect to the laser for appropriate correction of the heat impulse reply.

8. A method as in one of the preceding Claims, characterized in that
the duration of the subjection of the raw materials to laser power amounts to 5-50 msec.

9. A method as in one of the preceding Claims, characterized in that
a beam diameter of ≤ 2 mm is employed.

10. A method as in one of the preceding Claims, characterized in that
one or more single beams are employed of a power of ≤ 100 watt each,

11. A device for the performance of the method as in one of the preceding Claims, where the raw material to be identified is stationary, having a laser for acting briefly upon at least one place on the surface of the raw material to be identified, a temperature measuring device and a computer-supported evaluator device connected to the temperature measuring device, characterized in that for the detection of the heat impulse reply from the raw material (6, 14, 30) to be identified a thermocamera (8, 42) or an infrared line scanner (22) is provided, the measurement signals of which, gained from the pyrometric temperature measurement, are fed to the computer-supported evaluator device (10, 21) which in accordance with one of the preceding Claims evaluates the measurement signals with regard to temperature maxima and/or temperature distributions and/or cooling rates and prepares analysis signals by utilization of reference libraries or fuzzy logic or neuronal networks.

12. A device for the performance of the method as in one of the Claims 1 to 10, where the raw material to be identified is in motion, having a laser for acting briefly upon at least one place on the surface of the material to be identified, a device for transporting the raw materials which are to be examined, a temperature measuring device and a computer-supported evaluator device connected to the temperature measuring device, characterized in that the measurement signals gained from the pyrometric temperature measurement are fed to the computer-supported evaluator device (10, 21) which evaluates the measurement signals in accordance with one of the Claims 1 to 10 with regard to temperature maxima and/or temperature distributions and/or rates of cooling and prepares analysis signals by making use of reference libraries or fuzzy logic or neuronal networks, a sensor (18) being provided, which detects the entry into a test section of a raw material (14), as well as a trigger signal transmitter (20) which is controlled by the output signal from the sensor and is connected to the laser (12) for controlling it in dependence upon the output signal from the sensor.

13. A device as in Claim 12, characterized in that
a laser (60) is employed in continuous wave operation.

14. A device as in Claim 12 or 13, characterized in that
a number of lasers are or a multiple beam divider (28, 38, 50) is arranged along or across the direction of motion of the raw material.

15. , A device as in Claim 14, characterized in that
a multiple beam divider (50) is employed for the generation of laser beans of different intensities.

16. A device as in one of the Claims 11 to 15, characterized in that
the laser light optics (12, 24, 28, 36, 38) are arranged underneath the raw material (6, 14, 30) for acting upon the surface of the raw material from below.

17. A device as in one of the Claims 11 to 16, characterized in that
a CO₂-laser is used as the laser.

18. A device as in one of the Claims 11 to 17, characterized in that
a number of lasers of different wavelengths are employed.

## Revendications

1. Procédé d'identification matérielle de matériaux, dans lequel les matériaux sont frappés par de l'énergie laser et on détecte le rayonnement thermique des matériaux après achèvement de l'échauffement et on évalue les propriétés optiques et thermiques spécifiques aux matériaux,
caractérisé en ce qu'au moins un emplacement de la surface du matériau à identifier est frappé par l'énergie laser de manière ponctuelle et de courte durée et on détecte par des techniques de mesure localement et/ou temporairement la réponse impulsionnelle de chaleur du matériau matériellement caractéristique pour la zone superficielle frappée et on l'évalue en ce qui concerne le type de matériau, par le fait qu'on mesure la température maximale, qui représente une mesure de l'absorption dépendant de la longueur d'onde ou respectivement de la profondeur de pénétration optique du matériau, directement après l'échauffement dû au laser et/ou la répartition de chaleur, qui est une mesure de l'absorption de rayonnement et de la répartition d'énergie du faisceau laser sur la surface du matériau, directement après l'irradiation laser et/ou la répartition de chaleur après un temps de refroidissement déterminé après achèvement de l'irradiation laser en tant que mesure de la conductibilité thermique du matériau à identifier en tenant compte de la répartition de température mise en oeuvre directement après l'irradiation laser et/ou des vitesses de refroidissement, qui représente une mesure de la conductibilité thermique en prenant en compte les gradients de température dans la matière du matériau engendrés directement après l'irradiation laser, la réponse impulsionnelle de chaleur étant dans tous les cas détectée par une mesure pyrométrique de température à l'aide d'une caméra thermique ou d'un scanner IR à balayage par lignes ou respectivement horizontal.

2. Procédé d'identification matérielle de matériaux, dans lequel les matériaux sont frappés par de l'énergie laser et on détecte le rayonnement thermique des matériaux après achèvement de l'échauffement et on évalue les propriétés optiques et thermiques spécifiques aux matériaux,
caractérisé en ce qu'au moins un emplacement de la surface du matériau à identifier est frappé par l'énergie laser de manière ponctuelle et de courte durée et on détecte par des techniques de mesure localement et/ou temporairement la réponse impulsionnelle de chaleur du matériau matériellement caractéristique pour la zone superficielle frappée et on l'évalue en ce qui concerne le type de matériau, par le fait qu'on mesure la température maximale, qui représente une mesure de l'absorption dépendant de la longueur d'onde ou respectivement de la profondeur de pénétration optique du matériau, directement après l'échauffement dû au laser et/ou la répartition de chaleur, qui est une mesure de l'absorption de rayonnement et de la répartition d'énergie du faisceau laser sur la surface du matériau, directement après l'irradiation laser et/ou la répartition de chaleur après un temps de refroidissement déterminé après achèvement de l'irradiation laser en tant que mesure de la conductibilité thermique du matériau à identifier en tenant compte de la répartition de température mise en oeuvre directement après l'irradiation laser et/ou des vitesses de refroidissement, qui représente une mesure de la conductibilité thermique en prenant en compte les gradients de température dans la matière du matériau engendrés directement après l'irradiation laser et que, dans tous les cas, pour l'identification de matériaux mis en mouvement dans un processus automatique, on commande le laser par un signal de déclenchement dépendant de l'entrée des matériaux à analyser dans un trajet de contrôle.

3. Procédé selon l'une des revendications précédentes, caractérisé en ce que la surface du matériau est frappée isochroniquement par plusieurs impulsions laser.

4. Procédé selon l'une des revendications précédentes,
caractérisé en ce que le matériau est frappé par plusieurs faisceaux laser d'intensités différentes.

5. Procédé selon l'une des revendications précédentes,
caractérisé en ce que le matériau est frappé par plusieurs faisceaux laser de longueurs d'onde différentes.

6. Procédé selon l'une des revendications précédentes,
caractérisé en ce que le matériau, pendant son déplacement à travers la zone d'action du laser, le long de son axe de déplacement, est frappé uniformément par le rayonnement laser.

7. Procédé selon l'une des revendications précédentes,
caractérisé en ce qu'on mesure additionnellement le gradient de température de la réponse impulsionnelle de chaleur et on l'évalue en fonction de l'angle de position de surface du matériau par rapport au laser pour une correction correspondante de la réponse impulsionnelle de chaleur.

8. Procédé selon l'une des revendications précédentes,
caractérisé en ce que la durée de frappe des matériaux par l'énergie laser est égale à 5 - 50 ms.

9. Procédé selon l'une des revendications précédentes,
caractérisé en ce qu'on utilise un diamètre de faisceau ≤ 2 mm.

10. Procédé selon l'une des revendications précédentes,
caractérisé en ce qu'on utilise un ou plusieurs faisceaux individuels ayant respectivement une puissance ≤ 100 watts.

11. Dispositif pour la mise en oeuvre du procédé selon l'une des revendications précédentes, dans le cas d'un matériau à identifier au repos, avec un laser pour une frappe de courte durée d'au moins un emplacement de la surface du matériau à identifier, un dispositif de mesure de température et un dispositif d'analyse assisté par ordinateur, relié au dispositif de mesure de température,
caractérisé en ce que, pour détecter la réponse impulsionnelle de chaleur du matériau (6, 14, 30) à identifier, on prévoit une caméra thermique (8, 42) ou un scanner IR à balayage par lignes (22), dont les signaux de mesure obtenus par la mesure de température pyrométrique sont amenés au dispositif d'analyse (10, 21) assisté par ordinateur, qui analyse les signaux de mesure selon l'une des revendications précédentes en ce qui concerne les maxima de température et/ou les répartitions de température et/ou les vitesses de refroidissement et approvisionne des signaux d'analyse en utilisant des bibliothèques de référence ou des logiques à algorithmes lâches ou des réseaux neuronaux.

12. Dispositif pour la mise en oeuvre du procédé selon l'une des revendications 1 à 10, dans le cas de matériaux à identifier mis en mouvement, avec un laser pour une frappe de courte durée d'au moins un emplacement de la surface du matériau à identifier, un dispositif de transport pour les matériaux à analyser, un dispositif de mesure de température et un dispositif d'analyse assisté par ordinateur, relié au dispositif de mesure de température,
caractérisé en ce que les signaux de mesure obtenus par la mesure de température pyrométrique sont amenés au dispositif d'analyse (10, 21) assisté par ordinateur, qui analyse les signaux de mesure selon l'une des revendications 1 à 10 en ce qui concerne les maxima de température et/ou les répartitions de température et/ou les vitesses de refroidissement et approvisionne des signaux d'analyse en utilisant des bibliothèques de référence ou des logiques à algorithmes lâches ou des réseaux neuronaux, un capteur (18) étant prévu qui détecte l'entrée d'un matériau (14) dans un trajet de contrôle, ainsi qu'un émetteur de signal de déclenchement (20) qui est commandé par le signal de sortie du capteur et est relié au laser (12) pour sa commande en dépendance du signal de sortie du capteur.

13. Dispositif selon la revendication 12,
caractérisé en ce qu'on utilise un laser (60) en régime continu.

14. Dispositif selon la revendication 12 ou 13,
caractérisé en ce que plusieurs lasers ou un séparateur de faisceaux multiples (28, 38, 50) pour le rayonnement laser sont ou respectivement est disposé(s) le long de la direction de déplacement ou transversalement à la direction de déplacement du matériau.

15. Dispositif selon la revendication 14,
caractérisé en ce qu'on utilise un émetteur de rayonnement multiple (50) pour générer des faisceaux laser d'intensités différentes.

16. Dispositif selon l'une des revendications 11 à 15,
caractérisé en ce que l'optique du laser (12, 24, 28, 36, 38) est disposée en-dessous du matériau (6, 14, 30) pour frapper la surface du matériau par en-dessous.

17. Dispositif selon l'une des revendications 11 à 16,
caractérisé en ce qu'un laser CO₂ est mis en oeuvre en tant que laser.

18. Dispositif selon l'une des revendications 11 à 17,
caractérisé en ce que plusieurs lasers avec diverses longueurs d'onde sont utilisés.
